# EUROPÄISCHE PATENTANMELDUNG

(11) **EP 0 963 975 A1**
(43) Veröffentlichungstag der Anmeldung: **15.12.1999**
(21) Anmeldenummer: 99111282.2
(22) Anmeldetag: 10.06.1999
(51) Int. Cl.: C07C 209/16, B01J 23/755, B01J 23/74

(54) **Verfahren zur Herstellung von Aminen**

(30) Priorität: 12.06.1998 DE 19826396
(71) Anmelder: BASF AKTIENGESELLSCHAFT, 67056 Ludwigshafen (DE)
(72) Erfinder: Wulff-Döring, Joachim, Dr., 67227 Frankenthal (DE); Hesse, Michael, Dr., 67549 Worms (DE); Melder, Johann-Peter, Dr., 67459 Böhl-Iggelheim (DE); Buskens, Philipp, Dr., 23204 Hoogstraaten (BE); Voit, Guido, Dr., 67251 Freinsheim (DE); Funke, Frank, Dr., 67117 Limburgerhof (DE)

(57) **Zusammenfassung**

Verfahren zur Herstellung von Aminen durch Umsetzung von primären oder sekundären Alkoholen mit Stickstoffverbindungen, ausgewählt aus der Gruppe Ammoniak, primäre und sekundäre Amine, bei Temperaturen von 80 bis 250°C und Drücken von 0,1 bis 40 MPa mit Wasserstoff in Gegenwart eines Katalysators enthaltend Zirkonium, Kupfer, Cobalt und Nickel, dadurch gekennzeichnet, daß die katalytisch aktive Masse
- 22 bis 40 Gew.-%: sauerstoffhaltige Verbindungen des Zirkoniums, berechnet als ZrO₂,
- 1 bis 30 Gew.-%: sauerstoffhaltige Verbindungen des Kupfers, berechnet als CuO,
- 15 bis 50 Gew.-%: sauerstoffhaltige Verbindungen des Nickels, berechnet als NiO, wobei das Molverhältnis von Nickel zu Kupfer größer 1 ist,
- 15 bis 50 Gew.-%: sauerstoffhaltige Verbindungen des Cobalts, berechnet als CoO,
- 0 bis 10 Gew.-%: sauerstoffhaltige Verbindungen des Aluminiums und/oder Mangans, berechnet als Al₂O₃ bzw. MnO₂,
und keine sauerstoffhaltigen Verbindungen des Molybdäns enthält.

## Beschreibung

Die vorliegende Erfindung betrifft neue Katalysatoren enthaltend Zirkonium, Kupfer, Cobalt und Nickel sowie die Verwendung dieser Katalysatoren zur Aminierung von primären oder sekundären Alkoholen in Gegenwart von Wasserstoff bei erhöhter Temperatur und erhöhtem Druck.

Die Erfindung betrifft weiterhin ein Verfahren zur Herstellung von Aminen durch Umsetzung von primären oder sekundären Alkoholen mit Stickstoffverbindungen, ausgewählt aus der Gruppe Ammoniak, primäre und sekundäre Amine, bei Temperaturen von 80 bis 250°C und Drücken von 0,1 bis 40 MPa mit Wasserstoff in Gegenwart eines Katalysators enthaltend Zirkonium, Kupfer, Cobalt und Nickel.

Aus DE-A-19 53 263 ist es bekannt, Amine durch die hydrierende Aminierung der entsprechenden Alkohole an Cobalt, Nickel und Kupfer enthaltenden Katalysatoren herzustellen. Als Trägermaterial wird in diesen Katalysatoren Aluminiumoxid oder Siliciumdioxid verwendet. Mit diesen Katalysatoren lassen sich bei hohen Temperaturen und Drücken gute Umsätze erzielen, bei tieferen Temperaturen und Drücken geht der Umsatz und die Selektivität stark zurück.

Aus der EP-A-254 335 sind Ni-Co-Ru-Katalysatoren auf Aluminiumoxid- oder Siliciumdioxid-Trägern, welche zusätzlich noch Halogenide in ihrer aktiven Masse enthalten, zur hydrierenden Aminierung von Alkoholen bekannt. Mit diesen Katalysatoren werden gemäß der beschriebenen Ausführungsbeispiele nur Umsätze von maximal 61 % erzielt.

Aus der US-A-4 151 204 sind Katalysatoren zur Herstellung von Aminoalkoholen bekannt, die aus einem Metall wie Cobalt, Nickel oder Kupfer, bevorzugt aus Nickel oder Cobalt bestehen, und gegebenenfalls mit geringen Mengen an Zirkonium dotiert sind, wobei das Zirkonium bezüglich des Nickels oder Cobalts in einem Atomverhältnis von 0,001 : 1 bis 0,3 : 1 zugesetzt wird. Höhere Zirkoniumgehalte führen zu Nebenreaktionen wie der Zersetzung der Produkte (loc. cit.: Spalte 3, Zeilen 62-63, Zeilen 67-68, und Spalte 4, Zeilen 1-2).

Aus der EP-A-382 049 sind Katalysatoren, die sauerstoffhaltige Zirkonium-, Kupfer-, Cobalt- und Nickelverbindungen enthalten, und Verfahren zur hydrierenden Aminierung von Alkoholen bekannt.

Der bevorzugte Zirkoniumoxidgehalt dieser Katalysatoren beträgt 70 bis 80 Gew.-% (loc. cit.: Seite 2, letzter Absatz; Seite 3, 3. Absatz; Beispiele). Diese Katalysatoren zeichnen sich zwar durch eine gute Aktivität und Selektivität aus, besitzen allerdings verbesserungswürdige Standzeiten.

Aus der EP-A-696 572 und der EP-A-697 395 sind Nickel-, Kupfer-, Zirkoniumoxid und Molybdänoxid enthaltende Katalysatoren zur katalytischen Aminierung von Alkoholen mit Stickstoffverbindungen und Wasserstoff bekannt. Mit diesen Katalysatoren werden zwar hohe Umsätze erzielt, aber es bilden sich Nebenprodukte (z.B. Ethylamin), die selbst bzw. deren Folgeprodukte in der Aufarbeitung stören.

Aus der EP-A-514 692 sind Kupfer-, Nickel- und/oder Cobalt-, Zirkonium- und/oder Aluminiumoxid enthaltende Katalysatoren zur katalytischen Aminierung von Alkoholen in der Gasphase mit Ammoniak oder primären Aminen und Wasserstoff bekannt. Diese Patentanmeldung lehrt, daß bei diesen Katalysatoren das Atomverhältnis von Nickel zu Kupfer 0,1 bis 1,0, bevorzugt 0,2 bis 0,5 (vergl. loc. cit.: Beispiel 1) betragen muß, da ansonsten bei der Aminierung von Alkoholen in erhöhtem Maße ausbeutemindernde Nebenprodukte auftreten (loc. cit.: Beispiele 6 und 12). Als Träger wird bevorzugt Aluminiumoxid (loc. cit.: Beispiele 1 bis 5 und 7 bis 11) verwendet.

Die ältere deutsche Patentanmeldung Nr. 19742911.4 beschreibt ein Verfahren zur Herstellung von Aminen aus Alkoholen und Stickstoffverbindungen unter Verwendung eines Katalysators, dessen katalytisch aktive Masse sauerstoffhaltige Verbindungen des Zirkoniums, Kupfers und Nickels und keine sauerstoffhaltigen Verbindungen des Cobalts oder Molybdäns enthält.

Der vorliegenden Erfindung lag die Aufgabe zugrunde, die Wirtschaftlichkeit bisheriger Verfahren zur hydrierenden Aminierung von Alkoholen zu verbessern und den zuvor genannten Nachteilen abzuhelfen. Es sollten Katalysatoren gefunden werden, die es erlauben, die hydrierende Aminierung von Alkoholen mit hohem Umsatz, guter Ausbeute, Selektivität und Katalysatorstandzeit durchzuführen.

Dementsprechend wurden Katalysatoren gefunden, deren aktive Masse neben 22 bis 40 Gew.-% sauerstoffhaltigen Verbindungen des Zirkoniums, berechnet als ZrO₂, 1 bis 30 Gew.-% sauerstoffhaltige Verbindungen des Kupfers, berechnet als CuO, 15 bis 50 Gew.-% sauerstoffhaltige Verbindungen des Nickels, berechnet als NiO, wobei das Molverhältnis von Nickel zu Kupfer größer 1 ist, 15 bis 50 Gew.-% sauerstoffhaltige Verbindungen des Cobalts, berechnet als CoO, 0 bis 10 Gew.-% sauerstoffhaltige Verbindungen des Aluminiums und/oder Mangans, berechnet als Al₂O₃ bzw. MnO₂, und keine sauerstoffhaltigen Verbindungen des Molybdäns enthält sowie deren vorteilhafte Verwendung zur Aminierung von primären oder sekundären Alkoholen in Gegenwart von Wasserstoff bei erhöhter Temperatur und erhöhtem Druck.

Des weiteren wurde ein verbessertes Verfahren zur Herstellung von Aminen durch Umsetzung von primären oder sekundären Alkoholen mit Stickstoffverbindungen, ausgewählt aus der Gruppe Ammoniak, primäre und sekundäre Amine, bei Temperaturen von 80 bis 250°C und Drücken von 1 bis 40 MPa mit Wasserstoff in Gegenwart Katalysators enthaltend Zirkoniun, Kupfer, Cobalt und Nickel gefunden, welches dadurch gekennzeichnet ist, daß man als Katalysator einen der in den Ansprüchen definierten Katalysatoren verwendet.

Im allgemeinen werden die erfindungsgemäßen Katalysatoren bevorzugt in Form von Vollkatalysatoren eingesetzt. Mit dem Begriff "Vollkatalysator" wird ein Katalysator bezeichnet, der im Gegensatz zu einen Trägerkatalysator nur aus katalytisch aktiver Masse besteht. Vollkatalysatoren können dergestalt eingesetzt werden, daß man die katalytisch aktive, zu Pulver vermahlene Masse in das Reaktionsgefäß einbringt oder, daß man die katalytisch aktive Masse nach Mahlung, Vermischung mit Formhilfsmitteln, Formung und Temperung als Katalysatorformkörper - beispielsweise als Kugeln, Zylinder, Ringe, Spiralen - im Reaktor anordnet.

Die Konzentrationsangaben, jeweils berechnet als ZrO₂, CuO, NiO, CoO, Al₂O₃ und MnO₂ nach der letzten Wärmebehandlung des Katalysators und vor dessen Reduktion mit Wasserstoff, beziehen sich jeweils - falls nicht anders angegeben - auf die katalytisch aktive Masse des Katalysators.

Die katalytisch aktive Masse des Katalysators ist als die Summe der Massen der katalytisch aktiven Bestandteile definiert und enthält im wesentlichen die katalytisch aktiven Bestandteile Zirkonium, Kupfer, Nickel, Cobalt sowie optional Aluminium und/oder Mangan.

Die Summe der katalytisch aktiven Bestandteile, berechnet als ZrO₂, CuO, NiO, CoO, Al₂O₃ und MnO₂, beträgt üblicherweise 70 bis 100 Gew.-%, bevorzugt 80 bis 100 Gew.-%, besonders bevorzugt 90 bis 100 Gew.-%, ganz besonders bevorzugt 100 Gew.-%.

Im allgemeinen beträgt der Zirkoniumoxidgehalt der erfindungsgemäßen Katalysatoren 22 bis 40 Gew.-%, bevorzugt 25 bis 40 Gew.-%, besonders bevorzugt 25 bis 35 Gew.-%.

Der Gehalt der katalytisch aktiven Masse an sauerstoffhaltigen Verbindungen des Aluminiums und/oder Mangans, berechnet als Al₂O₃ und/oder MnO₂, kann bis zu 10 Gew.-% betragen, wobei das Gewichteverhältnis von Zirkonium, berechnet als ZrO₂, zu Aluminium und/oder Mangan, berechnet als Al₂O₃ und/oder MnO₂, mindestens 2,2, bevorzugt mindestens 2,5, besonders bevorzugt mindestens 5, beträgt.

Die anderen Komponenten berechnet als Nickeloxid, Cobaltoxid und Kupferoxid sind im allgemeinen insgesamt in Mengen von 31 bis 78 Gew.-%, bevorzugt 44 bis 75 Gew.-%, besonders bevorzugt 55 bis 75 Gew.-%, in der katalytisch aktiven Masse enthalten, wobei das Molverhältnis von Nickel zu Kupfer größer 1 ist.

Die Katalysatoren enthalten in ihrer katalytisch aktiven Masse
- 22 bis 40 Gew.-%,: bevorzugt 25 bis 40 Gew.-%, sauerstoffhaltige Verbindungen des Zirkoniums,
- 1 bis 30 Gew.-%,: bevorzugt 2 bis 25 Gew.-%, besonders bevorzugt 5 bis 15 Gew.-%, sauerstoffhaltige Verbindungen des Kupfers,
- 15 bis 50 Gew.-%,: bevorzugt 21 bis 45 Gew.-%, besonders bevorzugt 25 bis 40 Gew.-%, sauerstoffhaltige Verbindungen des Nickels, wobei das Molverhältnis von Nickel zu Kupfer größer 1, bevorzugt größer 1,2, besonders bevorzugt 1,8 bis 8,5, ist,
- 15 bis 50 Gew.-%: sauerstoffhaltige Verbindungen des Cobalts, berechnet als CoO,
- 0 bis 10 Gew.-%: sauerstoffhaltige Verbindungen des Aluminiums und/oder Mangans, wobei das Gewichtsverhältnis von Zirkonium, berechnet als ZrO₂, zu Aluminium und/oder Mangan, berechnet als Al₂O₃ und/oder MnO₂, bevorzugt mindestens 2,5 beträgt, ganz besonders bevorzugt 0 Gew.-% sauerstoffhaltige Verbindungen des Aluminiums und/oder Mangans,
und keine sauerstoffhaltigen Verbindungen des Molybdäns.

Zur Herstellung der Vollkatalysatoren sind verschiedenerlei Verfahrensweisen möglich. Sie sind beispielsweise durch Peptisieren pulvriger Mischungen der Hydroxide, Carbonate, Oxide und/oder anderer Salze der Komponenten Zirkonium, Nickel, Cobalt und Kupfer mit Wasser und nachfolgendes Extrudieren und Tempern der so erhaltenen Masse erhältlich.

Im allgemeinen werden zur Herstellung der erfindungsgemäßen Katalysatoren jedoch Fällungsmethoden angewandt. So können sie beispielsweise durch eine gemeinsame Fällung der Nickel-, Cobalt- und Kupferkomponenten aus einer diese Elemente enthaltenden, wässrigen Salzlösung mittels Mineralbasen in Gegenwart einer Aufschlämmung einer schwerlöslichen, sauerstoffhaltigen Zirkoniumverbindung und anschließendes Waschen, Trocknen und Calcinieren des erhaltenen Präzipitats erhalten werden. Als schwerlösliche, sauerstoffhaltige Zirkoniumverbindungen können beispielsweise Zirkoniumdioxid, Zirkoniumoxidhydrat, Zirkoniumphosphate, -borate und -silikate Verwendung finden. Die Aufschlämmungen der schwerlöslichen Zirkoniumverbindungen können durch Suspendieren feinkörniger Pulver dieser Verbindungen in Wasser unter kräftigem Rühren hergestellt werden. Vorteilhaft werden diese Aufschlämmungen durch Ausfällen der schwerlöslichen Zirkoniumnverbindungen aus wässrigen Zirkoniumsalzlösungen mittels Mineralbasen erhalten.

Bevorzugt werden die erfindungsgemäßen Katalysatoren über eine gemeinsame Fällung (Mischfällung) aller ihrer Komponenten hergestellt. Dazu wird zweckmäßigerweise eine die Katalysatorkomponenten enthaltende, wässrige Salzlösung in der Wärme und unter Rühren so lange mit einer wässrigen Mineralbase, insbesondere einer Alkalimetallbase - beispielsweise Natriumcarbonat, Natriumhydroxid, Kaliumcarbonat oder Kaliumhydroxid - versetzt, bis die Fällung vollständig ist. Die Art der verwendeten Salze ist im allgemeinen nicht kritisch: Da es bei dieser Vorgehensweise vornehmlich auf die Wasserlöslichkeit der Salze ankommt, ist ein Kriterium ihre zur Herstellung dieser verhältnismäßig stark konzentrierten Salzlösungen erforderliche, gute Wasserlöslichkeit. Es wird als selbstverständlich erachtet, daß bei der Auswahl der Salze der einzelnen Komponenten natürlich nur Salze mit solchen Anionen gewählt werden, die nicht zu Störungen führen, sei es, indem sie unerwünschte Fällungen verursachen oder indem sie durch Komplexbildung die Fällung erschweren oder verhindern.

Die bei diesen Fällungsreaktionen erhaltenen Niederschläge sind im allgemeinen chemisch uneinheitlich und bestehen u.a. aus Mischungen der Oxide, Oxidhydrate, Hydroxide, Carbonate und unlöslichen und basischen Salze der eingesetzten Metalle. Es kann sich für die Filtrierbarkeit der Niederschläge als günstig erweisen, wenn sie gealtert werden, d.h. wenn man sie noch einige Zeit nach der Fällung, gegebenenfalls in Wärme oder unter Durchleiten von Luft, sich selbst überläßt.

Die nach diesen Fällungsverfahren erhaltenen Niederschläge werden wie üblich zu den erfindungsgemäßen Katalysatoren weiterverarbeitet. Nach dem Waschen werden sie im allgemeinen bei 80 bis 200°C, vorzugsweise bei 100 bis 150°C, getrocknet und danach calciniert. Die Calcinierung wird im allgemeinen bei Temperaturen zwischen 300 und 800°C, vorzugsweise bei 400 bis 600°C, insbesondere bei 450 bis 550°C ausgeführt.

Nach der Calcinierung wird der Katalysator zweckmäßigerweise konditioniert, sei es, daß man ihn durch Vermahlen auf eine bestimmte Korngröße einstellt oder daß man ihn nach seiner Vermahlung mit Formhilfsmitteln wie Graphit oder Stearinsäure vermischt, mittels einer Tablettenpresse zu Formlingen verpreßt und tempert. Die Tempertemperaturen entsprechen dabei im allgemeinen den Temperaturen bei der Calcinierung.

Die auf diese Weise hergestellten Katalysatoren enthalten die katalytisch aktiven Metalle in Form eines Gemisches ihrer sauerstoffhaltigen Verbindungen, d.h. insbesondere als Oxide und Mischoxide.

Die auf diese Weise hergestellten Katalysatoren werden als solche gelagert und ggf. gehandelt. Vor ihrem Einsatz als Katalysatoren zur hydrierenden Aminierung von Alkoholen werden sie üblicherweise vorreduziert. Sie können jedoch auch ohne Vorreduktion eingesetzt werden, wobei sie dann unter den Bedingungen der hydrierenden Aminierung durch den im Reaktor vorhandenen Wasserstoff reduziert werden. Zur Vorreduktion werden die Katalysatoren im allgemeinen zunächst bei 150 bis 200°C über einen Zeitraum von 12 bis 20 Stunden einer Stickstoff-Wasserstoff-Atmosphäre ausgesetzt und anschließend noch bis zu ca. 24 Stunden bei 200 bis 400°C in einer Wasserstoffatmosphäre behandelt. Bei dieser Vorreduktion wird ein Teil der in den Katalysatoren vorliegenden sauerstoffhaltigen Metallverbindungen zu den entsprechenden Metallen reduziert, so daß diese gemeinsam mit den verschiedenartigen Sauerstoffverbindungen in der aktiven Form des Katalysators vorliegen.

Amine der allgemeinen Formel I 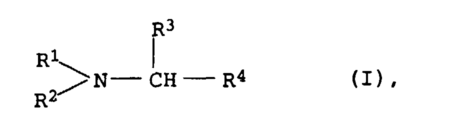 in der
- R¹ und R²: Wasserstoff, C₁- bis C₂₀-Alkyl, C₃- bis C₁₂-Cycloalkyl, Aryl, C₇- bis C₂₀-Aralkyl und C₇- bis C₂₀-Alkylaryl oder gemeinsam (CH₂)ⱼ-X-(CH₂)ₖ,
- R³ und R⁴: Wasserstoff, Alkyl, wie C₁- bis C₂₀₀-Alkyl, Cycloalkyl, wie C₃- bis C₁₂-Cycloalkyl, C₁- bis C₂₀-Hydroxyalkyl, durch Amino und/oder Hydroxy substituiertes C₁- bis C₂₀-Alkyl, Alkoxyalkyl, wie C₂- bis C₃₀-Alkoxyalkyl, Dialkylaminoalkyl, wie C₃- bis C₃₀-Dialkylaminoalkyl, Alkylaminoalkyl, wie C₂- bis C₃₀ -Alkylaminoalkyl, R⁵-(OCR⁶R⁷CR⁸R⁹)ₙ-(OCR⁶R⁷), Aryl, Heteroaryl, Aralkyl, wie C₇- bis C₂₀-Aralkyl, Heteroarylalkyl, wie C₄- bis C₂₀-Heteroarylalkyl, Alkylaryl, wie C₇- bis C₂₀-Alkylaryl, Alkylheteroaryl, wie C₄-bis C₂₀-Alkylheteroaryl und Y-(CH₂)ₘ-NR⁵-(CH₂)_{q} oder gemeinsam (CH₂)₁-X-(CH₂)ₘ oder
- R² und R⁴: gemeinsam (CH₂)₁-X-(CH₂)ₘ,
- R⁵, R¹⁰: Wasserstoff, C₁- bis C₄-Alkyl, C₇- bis C₄₀-Alkylphenyl,
- R⁶, R⁷, R⁸, R⁹: Wasserstoff, Methyl oder Ethyl,
- X: CH₂, CHR⁵, Sauerstoff oder NR⁵,
- Y: N(R¹⁰)₂, Hydroxy, C₂- bis C₂₀-Alkylaminoalkyl oder C₃- bis C₂₀-Dialkylaminoalkyl,
- n: eine ganze Zahl von 1 bis 30,
- j, k, l, m, q: eine ganze Zahl von 1 bis 4,
bedeuten, sind wirtschaftlich von besonderem Interesse. Das erfindungsgemäße Verfahren findet daher bevorzugt zur Herstellung der Amine I Anwendung, indem man primäre oder sekundäre Alkohole der allgemeinen Formel II

R⁴―CHR³―OH (II)

und Stickstoffverbindungen der allgemeinen Formel III 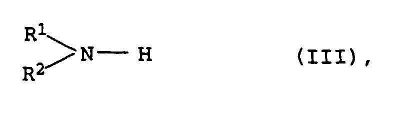 in denen R¹, R² sowie R³ und R⁴ die oben genannten Bedeutungen haben, umgesetzt.

Zur Herstellung des Amins I wird demnach rein formal ein Wasserstoffatom des Amins III durch den Alkylrest R⁴-CHR³- des Alkohols II unter Freisetzung von einem Moläquivalent Wasser ersetzt.

Das erfindungsgemäße Verfahren findet auch bevorzugt Anwendung bei der Herstellung von zyklischen Aminen der Formel IV 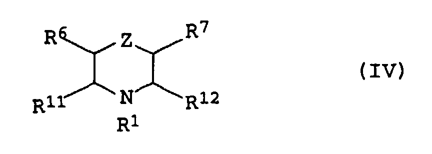 in der
- R¹¹ und R¹²: Wasserstoff, C₁- bis C₂₀-Alkyl, C₃- bis C₁₂-Cycloalkyl, Aryl, Heteroaryl, C₇- bis C₂₀-Aralkyl und C₇- bis C₂₀-Alkylaryl und
- Z: CH₂, CHR⁵, O, NR⁵ oder NCH₂CH₂OH bedeutet,
durch Umsetzung von Alkoholen der Formel V 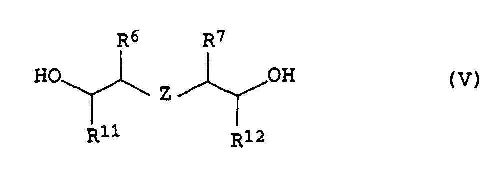 mit Ammoniak oder primären Aminen der Formel VI

R¹― NH₂ (VI).

Die Substituenten R¹, R²_{'} R³, R⁴, R⁵, R⁶, R⁷, R⁸, R⁹, R¹⁰, R¹¹, R¹², die Variablen X, Y, Z und die Indizes j, k, l, m, n und q in den Verbindungen I, II, III, IV, V und VI haben unabhängig voneinander folgende Bedeutungen:
R¹, R^{2,} R³, R⁴, R⁵, R⁶, R⁷, R⁸, R⁹, R¹⁰, R¹¹, R¹²,
   - Wasserstoff,
R³, R⁴
   - C₁- bis C₂₀₀-Alkyl, bevorzugt C₁- bis C₁₄-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, 2-Ethylhexyl, n-Decyl, 2-n-Propyl-n-heptyl, n-Tridecyl, 2-n-Butyl-n-nonyl und 3-n-Butyl-n-nonyl, besonders bevorzugt iso-Propyl, 2-Ethylhexyl, n-Decyl, 2-n-Propyl-n-heptyl, n-Tridecyl, 2-n-Butyl-n-nonyl und 3-n-Butyl-n-nonyl sowie bevorzugt C₄₀- bis C₂₀₀-Alkyl, wie Polybutyl, Polyisobutyl, Polypropyl, Polyisopropyl und Polyethyl, besonders bevorzugt Polybutyl und Polyisobutyl,
   - C₁- bis C₂₀-Hydroxyalkyl, bevorzugt C₁- bis C₈-Hydroxyalkyl, besonders bevorzugt C₁- bis C₄-Hydroxyalkyl, wie Hydroxymethyl, 1-Hydroxyethyl, 2-Hydroxyethyl, 1-Hydroxy-n-propyl, 2-Hydroxy-n-propyl, 3-Hydroxy-n-propyl und 1-Hydroxy-methylethyl,
   - durch Amino und/oder Hydroxy substituiertes C₁- bis C₂₀-Alkyl, bevorzugt durch Amino und/oder Hydroxy substituiertes C₁- bis C₈-Alkyl, besonders bevorzugt durch Amino und/oder Hydroxy substituiertes C₁- bis C₄-Alkyl, wie N-(Hydroxyethyl)aminoethyl und N-(Aminoethyl)aminoethyl,
   - C₂- bis C₃₀-Alkoxyalkyl, bevorzugt C₂- bis C₂₀-Alkoxyalkyl, besonders bevorzugt C₂- bis C₈-Alkoxyalkyl, wie Methoxymethyl, Ethoxymethyl, n-Propoxymethyl, iso-Propoxymethyl, n-Butoxymethyl, iso-Butoxymethyl, sec.-Butoxymethyl, tert.-Butoxymethyl, 1-Methoxy-ethyl und 2-Methoxyethyl, besonders bevorzugt C₂- bis C₄-Alkoxyalkyl, wie Methoxymethyl, Ethoxymethyl, n-Propoxymethyl, iso-Propoxymethyl, n-Butoxymethyl, iso-Butoxymethyl, sec.-Butoxymethyl, tert.-Butoxymethyl, 1-Methoxyethyl und 2-Methoxyethyl,
   - R⁵-(OCR⁶R⁷CR⁸R⁹)ₙ-(OCR⁶R⁷), bevorzugt R⁵-(OCHR⁷CHR⁹)ₙ-(OCR⁶R⁷), besonders bevorzugt R⁵-(OCH₂CHR⁹)ₙ-(OCR⁶R⁷),
   - C₃- bis C₃₀-Dialkylaminoalkyl, bevorzugt C₃- bis C₂₀- Dialkylaminoalkyl, besonders bevorzugt C₃- bis C₁₀- Dialkylaminoalkyl, wie Dimethylaminomethyl, Dimethylaminoethyl, Diethylaminoethyl, Di-n-propylaminoethyl und Di-iso-propylaminoethyl, (R⁵)₂N-(CH₂)_{q},
   - C₂- bis C₃₀-Alkylaminoalkyl, bevorzugt C₂- bis C₂₀- Alkylaminoalkyl besonders bevorzugt C₂- bis C₈- Alkylaminoalkyl, wie Methylaminomethyl, Methylaminoethyl, Ethylaminomethyl, Ethylaminoethyl und iso-Propylaminoethyl, (R⁵)HN-(CH₂)_{q},
   - Y-(CH₂)ₘ-NR⁵-(CH₂)_{q},
   - C₄- bis C₂₀-Heteroarylalkyl, wie Pyrid-2-yl-methyl, Furan-2-yl-methyl, Pyrrol-3-yl-methyl und Imidazol-2-yl-methyl,
   - C₄- bis C₂₀-Alkylheteroaryl, wie 2-Methyl-3-pyridinyl, 4,5-Dimethyl-imidazol-2-yl, 3-Methyl-2-furanyl und 5-Methyl-2-pyrazinyl,
   - Heteroaryl, wie 2-Pyridinyl, 3-Pyridinyl, 4-Pyridinyl, Pyrazinyl, Pyrrol-3-yl, Imidazol-2-yl, 2-Furanyl und 3-Furanyl,
R¹, R², R³, R⁴
   - C₃- bis C₁₂-Cycloalkyl, bevorzugt C₃- bis C₈-Cycloalkyl, wie Cyclopropyl, Cyclobutyl, Cyclopentyl, Cyclohexyl, Cycloheptyl und Cyclooctyl, besonders bevorzugt Cyclopentyl, Cyclohexyl und Cyclooctyl,
   - Aryl, wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl,
   - C₇- bis C₂₀-Alkylaryl, bevorzugt C₇- bis C₁₂-Alkylphenyl, wie 2-Methylphenyl, 3-Methylphenyl, 4-Methylphenyl, 2,4-Dimethylphenyl, 2,5-Dimethylphenyl, 2,6-Dimethylphenyl, 3,4-Dimethylphenyl, 3,5-Dimethylphenyl, 2,3,4-Trimethylphenyl, 2,3,5-Trimethylphenyl, 2,3,6-Trimethylphenyl, 2,4,6-Trimethylphenyl, 2-Ethylphenyl, 3-Ethylphenyl, 4-Ethylphenyl, 2-n-Propylphenyl, 3-n-Propylphenyl und 4-n-Propylphenyl,
   - C₇- bis C₂₀-Aralkyl, bevorzugt C₇- bis C₁₂-Phenylalkyl, wie Benzyl, 1-Phenethyl, 2-Phenethyl, 1-Phenyl-propyl, 2-Phenyl-propyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl,
   - R³ und R⁴ oder R² und R⁴ gemeinsam eine -(CH₂)ₗ-X-(CH₂)ₘ-Gruppe.
R¹, R²
   - C₁- bis C₂₀-Alkyl, bevorzugt C₁- bis C₈-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, besonders bevorzugt C₁- bis C₄-Alkyl wie Methyl, Ethyl, n-Propyl, iso-Propyl, n-Butyl, iso-Butyl, sec-Butyl und tert.-Butyl,
   - R¹ und R² gemeinsam eine -(CH₂)ⱼ-X-(CH₂)ₖ-Gruppe.
R⁵, R¹⁰
   - C₁- bis C₄-Alkyl, wie Methyl, Ethyl, n-Propyl, iso-Propyl, n-Butyl, iso-Butyl, sec.-Butyl. und tert.-Butyl, bevorzugt Methyl und Ethyl, besonders bevorzugt Methyl,
   - C₇- bis C₄₀-Alkylphenyl, wie 2-Methylphenyl, 3-Methylphenyl, 4-Methylphenyl, 2,4-Dimethylphenyl, 2,5-Dimethylphenyl, 2,6-Dimethylphenyl, 3,4-Dimethylphenyl, 3,5-Dimethylphenyl, 2-, 3-, 4-Nonylphenyl, 2-, 3-, 4-Decylphenyl, 2,3-, 2,4-, 2,5-, 3,4-, 3,5-Dinonylphenyl, 2,3-, 2,4-, 2,5-, 3,4- und 3,5-Didecylphenyl,
R⁶, R⁷, R⁸, R⁹
   - Methyl und Ethyl, bevorzugt Methyl,
R¹¹, R¹²
   - C₁- bis C₂₀-Alkyl, C₃- bis C₁₂-Cycloalkyl, Aryl, Heteroaryl, C₇- bis C₂₀-Aralkyl und C₇- bis C₂₀-Alkylaryl, jeweils wie oben definiert,
X
   - CH₂, CHR⁵, Sauerstoff oder NR⁵,
Y
   - N(R¹⁰)₂,
   - Hydroxy,
   - C₂- bis C₂₀-Alkylaminoalkyl, bevorzugt C₂- bis C₁₆-Alkylaminoalkyl, wie Methylaminomethyl, Methylaminoethyl, Ethylaminomethyl, Ethylaminoethyl und iso-Propylaminoethyl,
   - C₃- bis C₂₀-Dialkylaminoalkyl, bevorzugt C₃- bis C₁₆-Dialkylaminoalkyl, wie Dimethylaminomethyl, Dimethylaminoethyl, Diethylaminoethyl, Di-n-propylaminoethyl und Di-iso-propylaminoethyl,
Z
   - CH₂, CHR⁵, O, NR⁵ oder NCH₂CH₂OH,
j, l
   - eine ganze Zahl von 1 bis 4 wie 1, 2, 3 und 4, bevorzugt 2 und 3, besonders bevorzugt 2,
k, m, q
   - eine ganze Zahl von 1 bis 4 wie 1, 2, 3 und 4, bevorzugt 2, 3 und 4, besonders bevorzugt 2 und 3,
n
   - eine ganze Zahl von 1 bis 10, bevorzugt eine ganze Zahl von 1 bis 8 wie 1, 2, 3, 4, 5, 6, 7 oder 8, besonders bevorzugt eine ganze Zahl von 1 bis 6 wie 1, 2, 3, 4, 5 oder 6.

Als Alkohole eignen sich praktisch alle primären und sekundären aliphatischen Alkohole. Die aliphatischen Alkohole können geradkettig, verzweigt oder zyklisiert sein. Sekundäre Alkohole werden ebenso aminiert wie primäre Alkohole. Hinsichtlich der Kohlenstoffzahl der aminierbaren Alkohole gibt es praktisch keine Beschränkungen. Die Alkohole können ferner Substituenten tragen, welche sich unter den Bedingungen der hydrierenden Aminierung inert verhalten, beispielsweise Alkoxy-, Alkenyloxy- oder Alkylaminogruppen. Sollen mehrwertige Alkohole aminiert werden, so hat man es über die Steuerung der Reaktionsbedingungen in der Hand, Aminoalkohole, zyklische Amine oder mehrfach aminierte Produkte zu erhalten.

Die Aminierung von 1,4-Diolen führt je nach Wahl der Reaktionsbedingungen zu 1-Amino-4-hydroxy-, 1,4-Diamino-Verbindungen oder zu fünfgliedrigen Ringen mit einem Stickstoffatom.

Die Aminierung von 1,6-Diolen führt je nach Wahl der Reaktionsbedingungen zu 1-Amino-6-hydroxy-, 1,6-Diamino-Verbindungen oder zu siebengliedrigen Ringen mit einem Stickstoffatom.

Die Aminierung von 1,5-Diolen führt je nach Wahl der Reaktionsbedingungen zu 1-Amino-5-hydroxy-, 1,5-Diamino-Verbindungen oder zu sechsgliedrigen Ringen mit einem Stickstoffatom. Aus Diglykol kann demnach Monoaminodiglykol (= ADG = H₂N-CH₂CH₂-O-CH₂CH₂-OH), Diaminodiglykol oder besonders bevorzugt Morpholin erhalten werden. Aus Diethanolamin wird entsprechend besonders bevorzugt Piperazin erhalten. Aus Triethanolamin kann N-(2-Hydroxyethyl)-piperazin erhalten werden.

Bevorzugt werden beispielsweise die folgenden Alkohole aminiert:

Methanol, Ethanol, n-Propanol, Isopropanol, n-Butanol, iso-Butanol, n-Pentanol, n-Hexanol, 2-Ethylhexanol, Tridecanol, Stearylalkohol, Palmitylalkohol, Cyclopentanol, Cyclohexanol, Ethanolamin, n-Propanolamin, Isopropanolamin, n-Pentanolamin, n-Hexanolamin, Diethanolamin, Triethanolamin, N-Alkyldiethanolamine, Diisopropanolamin, N,N-Dimethylamino-ethanol, N,N-Diethylamino-ethanol, N,N-Di-n-propylamino-ethanol, N,N-Di-iso-propylamino-ethanol, N,N-Di-n-butylamino-ethanol, N,N-Di-iso-butylamino-ethanol, N,N-Di-sec.-butylamino-ethanol, N,N-Di-tert.-butylamino-ethanol, N,N-Dimethylamino-propanol, N,N-Diethylamino-propanol, N,N-Di-n-propylamino-propanol, N,N-Di-iso-propylamino-propanol, N,N-Di-n-butylamino-propanol, N,N-Di-iso-butylamino-propanol, N,N-Di-sec.-butylamino-propanol, N,N-Di-tert.-butylamino-propanol, 1-Dimethylamino-pentanol-4, 1-Diethylamino-pentanol-4, Ethylenglykol, 1,2-Propylenglykol, 1,3-Propylenglykol, Diglykol, 1,4-Butandiol, 1,5-Pentandiol, 1,6-Hexandiol, 2,2-Bis[4-hydroxycyclohexyl]propan, Methoxyethanol, Propoxyethanol, Butoxyethanol, Polyisobutylalkohole, Polypropylalkohole, Polyethylenglykolether, Polypropylenglykolether und Polybutylenglykolether. Die letztgenannten Polyalkylenglykolether werden bei der erfindungsgemäßen Umsetzung durch Umwandlung ihrer freien Hydroxylgruppen zu den entsprechenden Aminen umgewandelt.

Als Aminierungsmittel bei der hydrierenden Aminierung von Alkoholen können sowohl Ammoniak als auch primäre oder sekundäre, aliphatische oder cycloaliphatische oder aromatische Amine eingesetzt werden.

Bei Verwendung von Ammoniak als Aminierungsmittel werden die alkoholischen Hydroxylgruppen zunächst in freie Aminogruppen (-NH₂) umgewandelt. Die so gebildeten primären Amine können mit weiterem Alkohol zu den entsprechenden sekundären Aminen und diese wiederum mit weiterem Alkohol zu den entsprechenden, vorzugsweise symmetrischen, tertiären Aminen reagieren. Je nach Zusammensetzung des Reaktionsansatzes und je nach den angewandten Reaktionsbedingungen - Druck, Temperatur, Reaktionszeit - lassen sich auf diese Weise je nach Wunsch bevorzugt primäre, sekundäre oder tertiäre Amine darstellen.

Aus mehrwertigen Alkoholen lassen sich auf diese Weise durch intramolekulare hydrierende Aminierung zyklische Amine wie z.B. Pyrrolidine, Piperidine, Piperazine und Morpholine herstellen.

Ebenso wie Ammoniak lassen sich primäre oder sekundäre Amine als Aminierungsmittel verwenden.

Bevorzugt werden diese Aminierungsmittel zur Herstellung unsymmetrisch substituierter Di- oder Trialkylamine, wie Ethyldiisopropylamin und Ethyldicyclohexylamin verwendet. Bevorzugt werden beispielsweise die folgenden Mono- und Dialkylamine als Aminierungsmittel verwendet: Methylamin, Dimethylamin, Ethylamin, Diethylamin, n-Propylamin, iso-Propylamin, Diisopropylamin, n-Butylamin, s-Butylamin, iso-Butylamin, n-Pentylamin, s-Pentylamin, iso-Pentylamin, n-Hexylamin, s-Hexylamin, iso-Hexylamin und Cyclohexylamin.

Das Aminierungsmittel kann bezüglich der zu aminierenden alkoholischen Hydroxylgruppe in stöchiometrischer Menge eingesetzt werden. Bevorzugt wird jedoch mit einen Überschuß an Aminierungsmittel gearbeitet und zwar im allgemeinen mit einem mehr als 5molaren Überschuß pro Mol zu aminierender alkoholischer Hydroxylgruppe. Speziell Ammoniak wird im allgemeinen mit einem 1,5 bis 250-fachen, bevorzugt 5 bis 100-fachen, insbesondere 10 bis 80-fachen molaren Überschuß pro Mol umzusetzender alkoholischer Hydroxylgruppen eingesetzt. Höhere Überschüsse sowohl an Ammoniak als auch an primären oder sekundären Aminen sind möglich.

Der Wasserstoff wird der Reaktion im allgemeinen in einer Menge von 5 bis 400 l, bevorzugt in einer Menge von 50 bis 200 l pro Mol Alkoholkomponente zugeführt, wobei die Literangaben jeweils auf Normalbedingungen umgerechnet wurden (S.T.P.).

Die Umsetzung erfolgt im allgemeinen ohne zusätzliches Lösungsmittel. Bei der Umsetzung hochmolekularer hochviskoser oder bei Raumtemperatur fester Ausgangsverbindungen oder Produkte kann es vorteilhaft sein, ein unter den Reaktionsbedingungen inertes Lösungsmittel, wie Tetrahydrofuran, Dioxan, N-Methylpyrrolidon oder Ethylenglykoldimethylether mitzuverwenden.

Üblicherweise führt man die Umsetzung bei Temperaturen von 80 bis 300°C, bevorzugt 120 bis 230°C, besonders bevorzugt 130 bis 220°C durch. Im allgemeinen wird die Reaktion bei einem Druck von 0,1 bis 40 MPa ausgeführt. Bevorzugt werden jedoch Drücke von 1 bis 25 MPa, insbesondere von 3 bis 22 MPa, angewandt.

Die Anwendung höherer Temperaturen und eines höheren Gesamtdruckes ist möglich. Der Gesamtdruck im Reaktionsgefäß, welcher sich aus der Summe der Partialdrücke des Aminierungsmittels, der Alkoholkomponente und der gebildeten Reaktionsprodukte sowie ggf. des mitverwendeten Lösungsmittels bei den angegebenen Temperaturen ergibt, wird zweckmäßigerweise durch Aufpressen von Wasserstoff auf den gewünschten Reaktionsdruck eingestellt.

Es kann für die Selektivität des vorliegenden Verfahrens vorteilhaft sein, die Katalysatorformkörper im Reaktor mit inerten Füllkörpern zu vermischen, sie sozusagen zu "verdünnen". Der Anteil der Füllkörper in solchen Katalysatorzubereitungen kann 20 bis 80, besonders 30 bis 60 und insbesonders 40 bis 50 Volumenteile betragen.

Praktisch geht man bei der Durchführung im allgemeinen so vor, daß man dem Katalysator, der sich üblicherweise in einem bevorzugt von außen beheizten Festbettreaktor befindet, bei der gewünschten Reaktionstemperatur und dem gewünschten Druck den Alkohol und das Aminierungsmittel simultan zuführt. Dabei belastet man den Katalysator im allgemeinen mit 0,02 bis 3, bevorzugt 0,05 bis 2 und besonders bevorzugt mit 0,1 bis 1,6 l Alkohol pro Liter Katalysator und Stunde. Hierbei ist es zweckmäßig, die Reaktanten bereits vor der Zuführung in das Reaktionsgefäß zu erwärmen und zwar bevorzugt auf die Reaktionstemperatur.

Der Reaktor kann sowohl in der Sumpf- als auch in der Rieselfahrweise betrieben werden, d.h. man kann die Reaktanten sowohl von unten nach oben als auch von oben nach unten durch den Reaktor leiten. Es versteht sich von selbst, daß sich das Verfahren sowohl diskontinuierlich als auch kontinuierlich durchführen läßt. In beiden Fällen kann das überschüssige Aminierungsmittel zusammen mit dem Wasserstoff im Kreis geführt werden. Ist der Umsatz bei der Reaktion nicht vollständig, so kann das nicht umgesetzte Ausgangsmaterial ebenfalls in die Reaktionszone zurückgeführt werden.

Aus dem Reaktionsaustrag werden, nachdem dieser zweckmäßigerweise entspannt worden ist, das überschüssige Aminierungsmittel und der Wasserstoff entfernt und die erhaltenen Aminierungsprodukte durch Destillation, Flüssigextraktion oder Kristallisation gereinigt. Das überschüssige Aminierungsmittel und der Wasserstoff werden vorteilhaft wieder in die Reaktionszone zurückgeführt. Das gleiche gilt für die eventuell nicht oder nicht vollständig umgesetzte Alkoholkomponente.

Das im Zuge der Umsetzung gebildete Reaktionswasser wirkt sich im allgemeinen auf den Umsetzungsgrad, die Reaktionsgeschwindigkeit, die Selektivität und die Katalysatorstandzeit nicht störend aus und wird deshalb zweckmäßigerweise erst bei der destillativen Aufarbeitung des Reaktionsproduktes aus diesem entfernt.

Die erfindungsgemäß erhältlichen Amine eignen sich u. a. als Zwischenprodukte bei der Herstellung von Kraftstoffadditiven (US-A-3 275 554; DE-A-21 25 039 und DE-A-36 11 230), Tensiden, Arznei- und Pflanzenschutzmitteln sowie von Vulkanisationsbeschleunigern.

### Beispiele

### A. Katalysatorherstellung

### Herstellung von Katalysator A (erfindungsgemäß)

Eine wäserige Lösung aus Nickelnitrat, Kupfernitrat, Cobaltnitrat und Zirkoniumacetat, die 2,39 % NiO, 2,39 % CoO, 0,94 % CuO und 2,82 % ZrO₂ enthielt, wurde gleichzeitig in einem Rührgefäß in einem konstanten Strom mit einer 20 %igen wässrigen Natriumcarbonatlösung bei einer Temperatur von 70°C, so gefällt, daß der mit einer Glaselektrode gemessene pH-Wert von 7,0 aufrechterhalten wurde.

Die erhaltene Suspension wurde filtriert und der Filterkuchen mit vollentsalztem Wasser gewaschen bis die elektrische Leitfähigkeit des Filtrats ca. 20 mS betrug. Danach wurde der Filterkuchen bei einer Temperatur von 150°C in einem Trockenschrank oder einem Sprühtrockner getrocknet. Das auf diese Weise erhaltene Hydroxidcarbonatgemisch wurde nun bei einer Temperatur von 500°C über einen Zeitraum von 4 Stunden getempert.

Der so erhaltene Katalysator A hatte die Zusammensetzung:
28 Gew.-% Ni, berechnet als NiO,
28 Gew.-% Co, berechnet als CoO,
11 Gew.-% Cu, berechnet als CuO,
33 Gew.-% Zr, berechnet als ZrO₂.

Das Katalysatorpulver wurde mit 3 Gew.-% Graphit vermischt und zu 5 x 3 mm-Tabletten verformt.

### Herstellung von Katalysator B (für Vergleichsversuch nach DE-A-19 53 263)

Aluminiumoxidextrudate mit einem Strangdurchmesser von 4 mm wurden mit einer Lösung überstellt, die jeweils 5 Gew.-% Co und Ni und 2 Gew.-% Cu (berechnet als Metall) enthielt. Es handelte sich hierbei um eine Lösung der Metallnitrate.

Nach ca. 15 Minuten Tränkzeit wurden die Stränge bei 120°C getrocknet und danach bei 520°C getempert.

Danach wurde die Tränkung/Trocknung/Temperung wiederholt. Der erhaltene Katalysator B hatte die Zusammensetzung:
76 Gew.% Al, berechnet als Al₂O₃,
4 Gew.% Cu, berechnet als CuO,
10 Gew.% Co. berechnet als CoO,
10 Gew.% Ni berechnet als NiO.

### Herstellung von Katalysator C (für Vergleichsversuch nach EP-A-382 049)

Für Vergleichsversuche wurde ein Katalysator C gemäß der EP-A-382 049 wie folgt hergestellt. Eine wässrige Lösung aus Zirkonium-, Kupfer(II)-, Cobalt(II)- und Nickel-(II)-salzen wurde simultan mit wässriger Natriumcarbonatlösung einer Dichte von 1,208 kg/l in eine Fällungsapparatur gepumpt, in der sich frisch gefälltes, in Wasser suspendiertes Zirkoniumdioxid befand. Der pH-Wert der Lösung wurde während der Fällung auf 6,0 konstant gehalten und nach Verbrauch der Mineralsalzlösung durch Zugabe von wässriger Natriumcarbonatlösung auf pH 7,5 angehoben. Der Niederschlag wurde gewaschen, bei 120°C zur Gewichtskonstanz getrocknet und bei 400°C bis zur Gewichtskonstanz calciniert. Die erhaltene Katalysatorrohmasse wurde vermahlen, mit 3 Gew.-% Graphit vermischt, tablettiert und nochmals bei 520°C 3 Stunden calciniert.

Der erhaltene Katalysator C hatte die Zusammensetzung:
76 Gew.% Zr, berechnet als ZrO₂,
4 Gew.% Cu, berechnet als CuO,
10 Gew.% Co, berechnet als CoO,
10 Gew.% Ni, berechnet als NiO.

### Herstellung von Katalysator D (für Vergleichsversuch nach EP-A-696 572)

Der Katalysator wurde analog Katalysator A hergestellt. Allerdings wurde nach der Waschung in den noch feuchten Filterkuchen, wie in EP-A-696 572 auf Seite 8 beschrieben, Ammoniunheptamolybdat eingearbeitet, so daß der erhaltene Katalysator D die folgende Zusammensetzung hatte.
50 Gew.-% Ni, berechnet als NiO,
17 Gew.-% Cu, berechnet als CuO,
1,5 Gew.-% Mo, berechnet als MoO₃,
31,5 Gew.-% Zr, berechnet als ZrO₂.

Das Katalysatorpulver wurde mit 3 Gew.-% Graphit vermischt und zu 5 x 3 mm-Tabletten verformt.

### Herstellung von Katalysator E (für Vergleichsversuch nach der deutschen Patentanmeldung Nr. 19742911.4)

Es wurde wie für die Herstellung des Katalysators A verfahren, mit dem Unterschied, daß eine wässrige Lösung aus Nickelnitrat, Kupfernitrat und Zirkoniumacetat, die 4,48 % NiO, 1,52 % CuO und 2,82 % ZrO₂ enthielt, zum Einsatz kam.

Der so erhaltene Katalysator E hatte die Zusammensetzung:
51 Gew.-% Ni, berechnet als NiO,
17 Gew.-% Cu, berechnet als CuO,
32 Gew.-% Zr, berechnet als ZrO₂.

Das Katalysatorpulver wurde mit 3 Gew.-% Graphit vermischt und zu 5 x 3 mm-Tabletten verformt.

### B. Herstellung von Morpholin durch hydrierende Aminierung von Diglykol

### Beispiel 1 (erfindungsgemäß)

Ein kontinuierlich betriebener Hochdruckreaktor wurde mit 500 cm³ Katalysator A gefüllt und stündlich mit 270 cm³ Diglykol und 500 cm³ flüssigem Ammoniak beschickt. Die Katalysatortemperatur wurde auf 180°C und der Druck im Reaktor, durch gleichzeitiges Aufpressen von Wasserstoff, auf 20 MPa eingestellt. Aus dem Reaktionsaustrag wurde nach dessen Entspannung uberschüssiges Ammoniak abdestilliert. Die gaschromatographische Analyse der gesammelten Reaktionsausträge ergab folgende Zusammensetzung:
Morpholin: 49 %
Aminodiglykol: 23 %
Diglykol: 22 % (entspricht einem Diglykol-Umsatz von 78 %)
Ethylamin: 900 ppm
Sonstige Verbindungen inkl. Wasser: < 6 %
Die Morpholin-Selektivität betrug 63 %.

### Beispiel 2 (Vergleichsbeispiel)

Bei der Durchführung des Versuchs wie in Beispiel 1 beschrieben unter Verwendung von Katalysator B mußte, unter ansonsten identischen Reaktionsbedingungen wie im Beispiel 1, eine Temperatur von 220°C eingestellt werden, um einen vergleichbaren Diglykol-Umsatz von wenigstens 58 % zu erzielen. Die gaschromatographische

Analyse der gesammelten Reaktionsausträge ergab folgende Zusammensetzung:
Morpholin: 17 %
Aminodiglykol: 37 %
Diglykol: 42 %
Sonstige Verbindungen inkl. Wasser: 4 %
Die Morpholin-Selektivität betrug 29 %.

Der Katalysator B war damit aufgrund der höheren erforderlichen Reaktionstemperatur verglichen mit dein erfindungsgemäßen Katalysator A deutlich weniger reaktiv und zeigte auch eine wesentlich niedrigere Morpholin-Selektivität.

### Beispiel 3 (Vergleichsbeispiel)

Bei der Durchführung des Versuchs wie in Beispiel 1 beschrieben unter Verwendung von Katalysator C mußte, unter ansonsten identischen Reaktionsbedingungen wie im Beispiel 1, eine Temperatur von 185°C eingestellt werden, um einen vergleichbaren Diglykol-Umsatz von wenigstens 64 % zu erzielen. Die gaschromatographische Analyse der gesammelten Reaktionsausträge ergab folgende Zusammensetzung:
Morpholin: 27 %
Aminodiglykol: 31 %
Diglykol: 36 %
Sonstige Verbindungen inkl. Wasser: 6 %
Die Morpholin-Selektivität betrug 42 %.

Dieser Katalysator war nach einer Versuchsdauer von wenigen Tagen zerfallen und deswegen nicht geeignet.

### Beispiel 4 (Vergleichsbeispiel)

Bei der Durchführung des Versuchs wie in Beispiel 1 beschrieben unter Verwendung von Katalysator D mußte, unter ansonsten identischen Reaktionsbedingungen wie im Beispiel 1, eine Temperatur von 190°C eingestellt werden, um einen vergleichbaren Diglykol-Umsatz von wenigstens 57 % zu erzielen. Die gaschromatographische Analyse der gesammelten Reaktionsausträge ergab folgende Zusammensetzung:
Morpholin: 21 %
Aminodiglykol: 30 %
Diglykol: 43 %
Ethylamin: 5200 ppm
Sonstige Verbindungen inkl. Wasser: < 6 %
Die Morpholin-Selektivität betrug 37 %.

Für die Reaktion zeigte der Katalysator D eine wesentlich niedrigere Morpholin-Selektivität und weiterhin eine deutlich ausgeprägtere Bildung des störenden Ethylamins als der erfindungsgeinäße Katalysator A in Beispiel 1.

### Beispiel 5 (Vergleichsbeispiel)

Bei der Durchführung des Versuchs unter den gleichen Reaktionsbedingungen wie in Beispiel 1 beschrieben unter Verwendung von Katalysator E ergab die gaschromatographische Analyse der gesammelten Reaktionsausträge folgende Zusammensetzung:
Morpholin: 29 %
Aminodiglykol: 26 %
Diglykol: 40 %
Ethylamin: 1400 ppm
Sonstige Verbindungen inkl. Wasser: < 5 %
Die Morpholin-Selektivität betrug 48 %.

Für die Reaktion zeigte der Katalysator E eine wesentlich niedrigere Morpholin-Selektivität bei einem zugleich geringerem Diglykol-Umsatz und weiterhin eine ausgeprägtere Bildung des störenden Ethylamins als der erfindungsgemäße Katalysator A in Beispiel 1.

## Patentansprüche

1. Verfahren zur Herstellung von Aminen durch Umsetzung von primären oder sekundären Alkoholen mit Stickstoffverbindungen, ausgewählt aus der Gruppe Ammoniak, primäre und sekundäre Amine, bei Temperaturen von 80 bis 250°C und Drücken von 0,1 bis 40 MPa mit Wasserstoff in Gegenwart eines Katalysators enthaltend Zirkonium, Kupfer, Cobalt und Nickel, dadurch gekennzeichnet, daß die katalytisch aktive Masse des Katalysators vor dessen Reduktion mit Wasserstoff
22 bis 40 Gew.-% sauerstoffhaltige Verbindungen des Zirkoniums, berechnet als ZrO₂,
1 bis 30 Gew.-% sauerstoffhaltige Verbindungen des Kupfers, berechnet als CuO,
15 bis 50 Gew.-% sauerstoffhaltige Verbindungen des Nickels, berechnet als NiO, wobei das Molverhältnis von Nickel zu Kupfer größer 1 ist,
15 bis 50 Gew.-% sauerstoffhaltige Verbindungen des Cobalts, berechnet als CoO,
0 bis 10 Gew.-% sauerstoffhaltige Verbindungen des Aluminiums und/oder Mangans, berechnet als Al₂O₃ bzw. MnO₂,
und keine sauerstoffhaltigen Verbindungen des Molybdäns enthält.

2. Verfahren nach Anspruch 1, dadurch gekennzeichnet, daß die katalytisch aktive Masse des Katalysators vor dessen Reduktion mit Wasserstoff 25 bis 40 Gew.-% sauerstoffhaltige Verbindungen des Zirkoniums, berechnet als ZrO₂, 2 bis 25 Gew.-% sauerstoffhaltige Verbindungen des Kupfers, berechnet als CuO, 21 bis 45 Gew.-% sauerstoffhaltige Verbindungen des Nickels, berechnet als NiO, wobei das Molverhältnis von Nickel zu Kupfer größer 1 ist, und 21 bis 45 Gew.-% sauerstoffhaltige Verbindungen des Cobalts, berechnet als CoO, enthält.

3. Verfahren nach den Ansprüchen 1 und 2 zur Herstellung von Aminen der allgemeinen Formel I 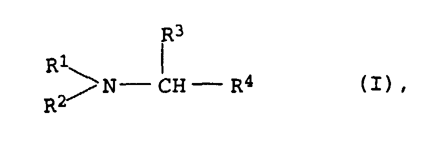 in der
R¹ und R² Wasserstoff, C₁- bis C₂₀-Alkyl, C₃- bis C₁₂-Cycloalkyl, Aryl, C₇- bis C₂₀-Aralkyl und C₇- bis C₂₀-Alkylaryl oder gemeinsam (CH₂)ⱼ-X-(CH₂)ₖ,
R³ und R⁴ Wasserstoff, Alkyl, Cycloalkyl, Hydroxyalkyl, durch Amino und/oder Hydroxy substituiertes Alkyl, Alkoxyalkyl, Dialkylaminoalkyl, Alkylaminoalkyl, R⁵-(OCR⁶R⁷CR⁸R⁹)ₙ-(OCR⁶R⁷), Aryl, Heteroaryl, Aralkyl, Heteroarylalkyl, Alkylaryl, Alkylheteroaryl und Y-(CH₂)ₘ-NR⁵-(CH₂)_{q} oder gemeinsam (CH₂)₁-X-(CH₂)ₘ oder
R² und R⁴ gemeinsam (CH₂)ₗ-X-(CH₂)ₘ,
R⁵, R¹⁰ Wasserstoff, C₁- bis C₄-Alkyl, C₇- bis C₄₀-Alkylphenyl,
R⁶, R⁷, R⁸, R⁹ Wasserstoff, Methyl oder Ethyl,
X CH₂, CHR⁵, Sauerstoff oder NR⁵,
Y N(R¹⁰)₂, Hydroxy, C₂- bis C₂₀-Alkylaminoalkyl oder C₃- bis C₂₀-Dialkylaminoalkyl,
n eine ganze Zahl von 1 bis 30,
j, k, l, m, q eine ganze Zahl von 1 bis 4,
bedeuten, aus primären oder sekundären Alkoholen der allgemeinen Formel II
R⁴―CHR³―OH (II)
und Stickstoffverbindungen der allgemeinen Formel III

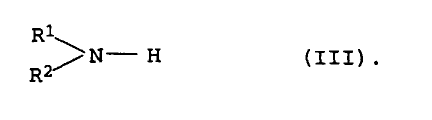

4. Verfahren nach den Ansprüchen 1 bis 3, dadurch gekennzeichnet, daß man die Umsetzung bei Temperaturen von 120 bis 230°C durchführt.

5. Verfahren nach den Ansprüchen 1 bis 4, dadurch gekennzeichnet, daß man die Umsetzung bei Drücken von 1 bis 25 MPa durchführt.

6. Verfahren nach den Ansprüchen 1 bis 4, dadurch gekennzeichnet, daß man die Umsetzung bei Drücken von 3 bis 22 MPa durchführt.

7. Katalysatoren, deren katalytisch aktive Masse
22 bis 40 Gew.-% sauerstoffhaltige Verbindungen des Zirkoniums, berechnet als ZrO₂,
1 bis 30 Gew.-% sauerstoffhaltige Verbindungen des Kupfers, berechnet als CuO,
15 bis 50 Gew.-% sauerstoffhaltige Verbindungen des Nickels, berechnet als NiO, wobei das Molverhältnis von Nickel zu Kupfer größer 1 ist,
15 bis 50 Gew.-% sauerstoffhaltige Verbindungen des Cobalts, berechnet als CoO,
0 bis 10 Gew.-% sauerstoffhaltige Verbindungen des Aluminiums und/oder Mangans, berechnet als Al₂O₃ bzw. MnO₂,
und keine sauerstoffhaltigen Verbindungen des Molybdäns enthält.

8. Katalysatoren nach Anspruch 7, deren katalytisch aktive Masse
25 bis 40 Gew.-% sauerstoffhaltige Verbindungen des Zirkoniums, berechnet als ZrO₂,
2 bis 25 Gew.-% sauerstoffhaltige Verbindungen des Kupfers, berechnet als CuO,
21 bis 45 Gew.-% sauerstoffhaltige Verbindungen des Nickels, berechnet als NiO, wobei das Molverhältnis von Nickel zu Kupfer größer 1 ist,
21 bis 45 Gew.-% sauerstoffhaltige Verbindungen des Cobalts, berechnet als CoO,
0 bis 10 Gew.-% sauerstoffhaltige Verbindungen des Aluminiums und/oder Mangans, berechnet als Al₂O₃ bzw. MnO₂,
und keine sauerstoffhaltigen Verbindungen des Molybdäns enthält.

9. Verwendung von Katalysatoren gemäß den Ansprüchen 7 und 8 zur Aminierung von primären oder sekundären Alkoholen mit Aminierungsmitteln, ausgewählt aus der Gruppe Ammoniak, primäre und sekundäre Amine, in Gegenwart von Wasserstoff bei erhöhter Temperatur und erhöhtem Druck.
